(19) Europäisches Patentamt
European Patent Office
Office européen des brevets

(11) Veröffentlichungsnummer: **0 080 119**
**B1**

(12) **EUROPÄISCHE PATENTSCHRIFT**

(45) Veröffentlichungstag der Patentschrift: **19.06.85**

(51) Int. Cl.⁴: **C 07 C 102/08**, C 07 C 103/48

(21) Anmeldenummer: **82110414.8**

(22) Anmeldetag: **11.11.82**

(54) **Verfahren zur Herstellung von N-Formyl-alpha-aminosäureestern.**

(30) Priorität: **19.11.81 DE 3145736**

(43) Veröffentlichungstag der Anmeldung: **01.06.83 Patentblatt 83/22**

(45) Bekanntmachung des Hinweises auf die Patenterteilung: **19.06.85 Patentblatt 85/25**

(84) Benannte Vertragsstaaten: **BE CH DE FR GB IT LI NL**

(56) Entgegenhaltungen:
**DE - B - 1 201 357**
**FR - A - 1 330 346**
**FR - A - 2 251 547**

**CHEMICAL ABSTRACTS, Band 62, Nr. 9, 26. April 1965, Spalten 9749h-9750a, Columbus, Ohio, USA, S.V. SHLYAPNIKOV et al.: "Comparative study of gas-chromatographic separation of the ester of N-acyl amino acids" & BIOKHIMIYA 29(6), 1076-1082, 1964**

(73) Patentinhaber: **BASF Aktiengesellschaft, Carl-Bosch-Strasse 38, D-6700 Ludwigshafen (DE)**

(72) Erfinder: **Boell, Walter, Dr., Hintergasse 35, D-6701 Dannstadt-Schauernheim (DE)**
Erfinder: **Kroener, Michael, Dr., Eislebener Weg 8, D-6800 Mannheim 42 (DE)**
Erfinder: **Beyer, Karl-Heinz, Dr., Knietschstrasse 6, D-6710 Frankenthal (DE)**
Erfinder: **Hertel, Dieter, Max-Reger-Weg 5, D-6906 Leimen (DE)**

**EP 0 080 119 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Es ist bekannt, zur Herstellung von N-Formylaminosäureestern Aminosäureester mit Ameisensäure bzw. einem Gemisch aus Ameisensäure und Essigsäureanhydrid umzusetzen. Bei diesem Verfahren ist es jedoch erforderlich, die nach der Veresterung der aminosauren Salze beispielsweise als Hydrochloride anfallenden Aminosäureester zuvor in die freien Verbindungen zu überführen.

Eine verbesserte Verfahrensweise wird in der DE-AS 12 01 357 beschrieben. Hierbei werden zunächst die Aminosäureesterhydrochloride hergestellt und diese mit Formamid umgesetzt. Es handelt sich dabei im Prinzip um eine Umamidierung, bei der festes Ammonchlorid gebildet wird.

Eine Variante dieser Verfahren wird von japanischen Autoren (Bull. Soc., Japan 1972, 45, 197-18) beschrieben. Hierbei werden die N-Formylaminosäureester durch Erhitzen der zugrunde liegenden Aminosäuren und Alkohole mit Ameisensäure im Autoklav auf 170° hergestellt.

Die beschriebenen Verfahren haben jedoch wesentliche Nachteile:

1. Ausgangsstoffe sind fertige Aminosäuren oder sogar deren Ester, die somit zuvor in einem eigenen Verfahren gewonnen werden müssen.
2. Nach der Veresterungsreaktion muß der überschüssige Alkohol und überschüssiger Chlorwasserstoff unter vermindertem Druck abgetrennt werden.
3. Nach der Formylierung mit Formamid muß ein Lösungsmittel zugesetzt und festes Ammonchlorid abgetrennt und gewaschen werden. Dieser Umstand ist technisch besonders unerwünscht, weil Feststoffanfall i. a. arbeitsintensiv ist.
4. Die angegebenen Ausbeuten liegen zwischen nur 35% und etwa 70% bezogen auf die eingesetzte Aminosäure.
5. Im Falle das japanischen Verfahrens muß darüber hinaus ein hoher Überschuß von Ameisensäure (3 mol/mol Aminosäure) entfernt werden.

Nach dem Vorschlag der FR-A-22 51 547 soll Iminodiacetonitril mit Methanol und Chlorwasserstoff zunächst zum Hydrochlorid des Iminodiacetodimethylesters umgesetzt werden, wobei nach Freisetzung der Base in einer Folgereaktion durch Formylierung mittels Chloral N,N'-Bis(acetomethyl)formamid gebildet wird. Dieses Verfahren muß mit großem Überschuß an Methanol und Chlorwasserstoff durchgeführt werden, wobei außerdem zahlreiche Trenn- und Reinigungsstufen vorgesehen sind. Die Gesamtausbeute an Wertprodukt ist gering (38% d. Th.).

Aufgabe der Erfindung ist die Schaffung eines vereinfachten Verfahrens, das die Handhabung hoher Überschußmengen an Reaktionsteilnehmern und i. a. auch das Auftreten von Feststoffen im Reaktionsablauf vermeidet und weitere Vorteile hat.

Die Erfindung macht sich die Tatsache zunutze, daß Aminosäuren im technischen Maßstab vorzugsweise nach dem Strecker'schen Verfahren aus Aldhyden, Blausäure und Ammoniak über die Cyanhydrine bzw. Aminonitrile gewonnen werden und geht daher aus von den Aminonitrilen.

Sie betrifft ein Verfahren zur Herstellung von N-Formylaminosäureestern durch Umsetzung von Aminonitrilen der allgemeinen Formel (I)

$$R^1-(CH_2)_n-\underset{\underset{CN}{|}}{\overset{\overset{NH_2}{|}}{C}}H \qquad (I)$$

wobei n den Wert 0 oder 1 und $R^1$ Wasserstoff oder, falls $n = 1$ ist, auch einen Alkylrest mit bis zu 4 C-Atomen, die Methoxygruppe oder die Methylthiomethylengruppe bedeutet, mit einem primären Alkohol in Gegenwart von Chlorwasserstoff und von mindestens soviel Wasser, wie bei der Umsetzung des Cyanhydrins mit Ammoniak zum Aminonitril nach der Strecker'schen Synthese frei wird.

Die Erfindung geht vorzugsweise aus von dem bei der Herstellung des Aminonitrils anfallenden Rohprodukt, dem zweckmäßig ein Teil des darin enthaltenen Ammoniaks durch eine Behandlung unter vermindertem Druck entzogen worden ist.

Eine vorteilhafte Arbeitsweise zur Entfernung der Hauptmenge an Ammoniak ist z. B. durch Gluud und Klempt in Ber. Ges. Kohletech. 5 (1950) S. 324 ff, beschrieben.

Das erhaltene ammoniakarme Aminonitril, das aber noch mindestens das bei der Vorreaktion entstandene Wasser enthält, kann in Gegenwart von Alkoholen der allgemeinen Formel

$$R^2-CH_2OH \qquad (II),$$

in der $R^2$ eine Alkylgruppe von bis zu 5, vorzugsweise bis zu 3 C-Atomen darstellt, mit gasförmigem Chlorwasserstoff gemäß Gleichung (1)

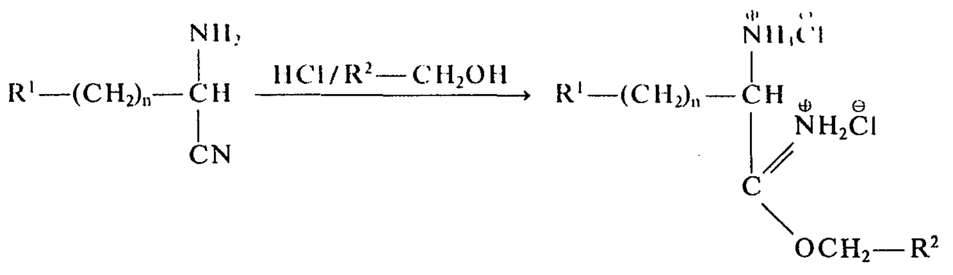


(I) (III)

(1)

über das Bishydrochlorid III in das Hydrochlorid der $\alpha$-Aminosäureester IV gemäß Gleichung (2)

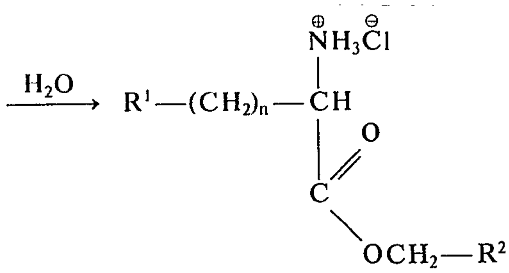


(III) (IV)

(2)

überführt werden.

Die Umsetzung läuft bei einer Temperatur bis zu 160°, vorteilhaft zwischen Raumtemperatur und 120°C ab. Die benötigte Alkoholmenge liegt zwischen 1,5 und 6 mol pro mol Aminonitril, vorzugsweise zwischen 2 und 4 mol, die HCl-Menge zwischen 2 und 4 mol, vorzugsweise zwischen 2,1 und 2,6 mol/mol Aminonitril, wobei der vom restlichen freien Ammoniak verbrauchte Anteil nicht berücksichtigt ist.

Es ist zweckmäßig, das Aminonitril bei möglichst niedriger Temperatur, z. B. unterhalb von etwa 10°C in die Reaktion einzubringen und in vielen Fällen empfiehlt sich auch die Aufbewahrung der wäßrigen Rohgemische bei einer solchen Temperatur.

Setz man nun ein solches Reaktionsgemisch — wie es anfällt — mit 1 bis 2 mol, vorzugsweise 1,1 bis 1,4 mol Formamid pro mol eingesetztem Aminonitril um, so erhält man in hoher Ausbeute und vorzüglicher Reinheit die formylierten $\alpha$-Aminosäureester V.

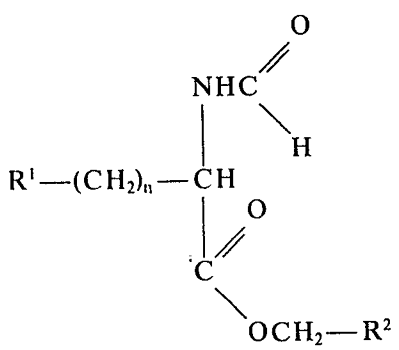

(V)

Für eine vollständige Formylierung ist eine Temperatur von über 100°C erforderlich, vorzugsweise 110 bis 130°C.

Die besonderen Vorzüge des Verfahrens liegen darin, daß

- zur Abtrennung von Ammonchlorid keine Lösungsmittel zugesetzt werden müssen;
- bei den Estern von Formyl-aminosäuren mit höheren Alkoholen Ammonchlorid nicht als Feststoff anfällt und somit auch nicht über Schleudern mit nachfolgendem Waschprozeß isoliert werden braucht, sondern als wäßrige Lösung abgetrennt werden kann;
- der etwa umgesetzte Chlorwasserstoff in besonders eleganter Weise durch das überschüssige Formamid in Ammonchlorid und Ameisensäureester überführt wird, der als Nebenausbeute gewonnen werden kann.

Das erfindungsgemäße Verfahren läßt sich außerdem vorteilhafterweise als sog. »Eintopfverfahren« aufziehen und liefert die z. T. neuen Formylester in ausgezeichneter Ausbeute. Z. B. wurde ohne besondere Optimierung eine Ausbeute bis zu 91,3% über alle Stufen erzielt; nach der fraktionierten Destillation ergab sich eine Reinheit von bis zu 99%. Einen Vergleich zu bekannten Verfahren zeigt die Tabelle 1.

Verwendung finden N-Formylaminosäureester u. a. für die Synthese von Isocyansäureestern z. B. nach Ugi, Angew. Chem. 77, 492 [1965] und für die Synthese von 5-Alkoxyoxazolen, wichtigen Zwischenprodukten für die Herstellung von Vitamin B6 [Übersicht von König und Böll, Chem. Ztg. 100, 107/8 (1976)].

Die Struktur der in den nachfolgenden Beispielen angegebenen Verbindungen (vgl. nachstehende Tabelle) wurde jeweils mit den Mitteln der kernmagnetischen Protonenresonanz-Analyse gesichert.

Tabelle 1

N-Formyl-α-aminosäureester

| Bei-spiele | 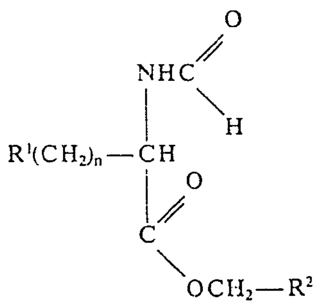 | | | $n_D^{20}$ | Reinheit Fl % (nach GC) | Ausbeute | | Elementaranalyse | | | | | | | |
|---|---|---|---|---|---|---|---|---|---|---|---|---|---|---|---|
| | | | | | | erfindungsgemäß (% d. Th.) | nach dem Stande der Technik (% d. Th.) | Berechnet (%) | | | | Gefunden (%) | | | |
| | | | | | | | | C | H | N | O | C | H | N | O |
| | $R_1$ | n | $R_2$ | | | | | | | | | | | | |
| 1 | H | 0 | $CH(CH_3)_2$ | 1,4500 | | 85 | | 52,8 | 8,23 | 8,8 | 30,2 | 52,9 | 8,2 | 8,6 | 29,6 |
| 2 | H | 1 | $CH_3$ | 1,4454 | 99,2 | 82,2 | 67,5[1]) 71[2]) | | | | | | | | |
| 3 | H | 1 | $C_2H_5$ | 1,4461 | 96,8 | 61,3 | | 52,8 | 8,23 | 8,8 | 30,2 | 52,0 | 8,0 | 9,3 | 30,3 |
| 4 | H | 1 | $CH(CH_3)_2$ | 1,4448 | 99,1 | 86,1 | | 55,5 | 8,73 | 8,09 | 27,7 | 55,3 | 8,8 | 8,2 | 27,3 |
| 5 | H | 1 | $CH_2CH_2CH_3$ | 1,4462 | 98,5 | 84 | 50[2]) | | | | | | | | |
| 6 | H | 1 | $CH_2{-}CH{-}(CH_3)_2$ | 1,4477 | 96,9 | 82 | | 57,7 | 9,1 | 7,5 | 25,7 | 57,5 | 9,1 | 8,0 | 24,9 |
| 7 | $CH_3$ | 1 | $CH(CH_3)_2$ | 1,4473 | 98,8 | 84 | | | | | | | | | |
| 8 | $CH_2CH_2CH_3$ | 1 | $CH(CH_3)_2$ | 1,4489 | 98,2 | 84 | | 61,4 | 9,8 | 6,5 | 22,3 | 61,2 | 9,7 | 6,8 | 21,7 |
| 9 | $CH(CH_3)_2$ | 1 | $CH_3$ | 1,4490 | 96 | 63 | 56[2]) | | | | | | | | |
| 10 | $CH(CH_3)_2$ | 1 | $CH(CH_3)_2$ | 1,4472 | 98,8 | 91,3 | | 61,4 | 9,8 | 6,5 | 22,3 | | | | |
| 11 | $OCH_3$ | 1 | $CH(CH_3)_2$ | 1,4504 | 98,5 | 79 | | 53,2 | 8,4 | 6,9 | 31,5 | 53,3 | 8,3 | 6,7 | 31,7 |
| 12 | $CH_2SCH_3$ | 1 | $CH(CH_3)_2$ | 1,4872 | | 70 | | 51,5 | 8,2 | 6,0 | 20,5 | 52,1 | 8,3 | 6,0 | 20,0 |
| | | | | | | | | S = 13,7 | | | | S = 14,0 | | | |

Lit. [1]): DE-OS 1 201 357 1964
Lit. [2]): Bull. Chem. Soc. Jap. 1972

## Beispiel 1

### N-Formylglycinisobutylester

160 g 74,3%iges wäßriges Glycinnitril (2 mol, enthaltend 2 mol Wasser und 0,1 mol Ammoniak) werden unter Kühlung einer Lösung von 170 g HCl-Gas (= 4,66 Mol) in 518 g (= 7,0 mol) Isobutanol so zugesetzt, daß eine Temperatur von 50° C nicht überschritten wird. Anschließend wird innerhalb von 4 Stunden auf 103° C aufgeheizt (Rückfluß). Man läßt 2 Stunden nachreagieren und setzt dann langsam 117 g (= 2,6 mol) Formamid zu. Nach 2,5 Stunden Nachreaktionszeit unter Rückfluß bei 109° läßt man auf 60° abkühlen, gibt 720 g Wasser zu und trennt 761 g organische Phase ab, die nach 3maliger Wäsche mit wäßriger Sodalösung und Destillation 270 g N-Formylglycin-isobutylester ergibt, d. s. 85% Ausbeute; $Kp_{0,5\ mbar}$ = 113 bis 114° C.

## Beispiel 2

### N-Formylalaninethylester

105,8 g auf 0° C gebrachtes 66,2%iges $\alpha$-Alaninnitril (1 mol enthaltend 3,7% Ammoniak/0,23 mol und 23,7% Wasser/1,4 mol) werden in eine Lösung von 91,2 g HCl (2,5 mol) in 184 g Ethanol (4,0 mol) wie im Beispiel 1 beschrieben zusetzt, innerhalb von 4 Stunden auf 78° aufgeheizt und weitere 4 Stunden bei dieser Temperatur gehalten. Nun werden 59 g Formamid (= 1,3 mol) in 1 Stunde zugesetzt. Dann wird 2 Stunden im Autoklaven bei 122° und einem autogenen Druck von 4,3 bar nachbehandelt. Bei Raumtemperatur werden 119,3 g festes Ammonchlorid (= 2,23 mol) isoliert und aus dem Filtrat nach fraktionierter Destillation 120,3 g 99,2%iger Ester d. s. 82,2% d. Theorie gewonnen. $Kp_{0,3\ mbar}$ = 95° C.

## Beispiel 3

### N-Formylalanin-n-propylester

106,3 g auf 0° gebrachtes wäßriges 65,9%iges $\alpha$-Alaninnitril (1 mol, enthaltend 4,1% $NH_3$/0,26 mol) werden einer Lösung von 94,4 g HCl (= 2,6 mol) in 211 g (= 3,5 mol) n-Propanol wie im Beispiel 1 beschrieben zugesetzt, anschließend wird innerhalb 2 Stunden auf 90° C erwärmt und weitere 2 Stunden bei 90° C gehalten. Nun werden 59 g Formamid (1,3 mol) innerhalb von 1 Stunde bei 90° zugesetzt und 2 Stunden bei 117° und dem sich einstellenden Druck von 2 bar nachbehandelt. Bei 60° C gibt man 363 g Wasser zu, trennt 319 g organischer Phase ab, wäscht 2mal mit je 40 g 3,4%iger Sodalösung und fraktioniert im Vakuum. Man erhält 100,5 g 97%igen Ester (GC), d. s. 61,3% d. Th.; $Kp_{0,5\ mbar}$ = 107 bis 110° C.

## Beispiel 4

### N-Formylalaninisobutylester; technischer Maßstab

1525 kg auf 0° C gebrachtes wäßriges 78,0%iges $\alpha$-Alaninnitril (17,0 kmol; enthaltend 1,9% $NH_3$/= 1,7 kmol) werden einen 12 000 l fassenden Kessel, der mit einer Lösung von 1 365 kg HCl (= 37,4 kmol) in 3585 kg (= 48,5 kmol) Isobutanol beschickt ist, so zugefahren, daß eine Temperatur von 50° C nicht überschritten wird. Nun wird 2 Stunden auf 110° C erhitzt, dann 956 kg Formamid (= 21,25 kmol) zugepumpt und weitere 2 Stunden am Rückfluß gehalten. Man läßt auf ca. 60° C abkühlen, setzt 6073 kg Wasser zu, läßt absitzen, trennt die organische Phase ab und wäscht 3mal mit Wasser. Man erhält 5761 kg N-Formylalaninisobutylester als 44%ige Lösung in wäßrigem Isobutanol, d. s. 86,1% Ausbeute; für die 99%ige Verbindung findet man $Kp_{1\ mbar}$ = 98 bis 99° C; $n_{20}^{D}$: 1,4448; $d^{25}$ : 1,022; Viskosität : 28 mPas.

## Beispiel 5

### N-Formylalanin-n-butylester

Analog Beispiel 3 werden 106,3 g 0° kaltes $\alpha$-Alaninnitril mit 222 g n-Butanol (= 3,0 mol) zur Reaktion gebracht und 2 Stunden bei 120° C formyliert. Nach der beschriebenen Aufarbeitung mit anschließender fraktionierter Destillation erhält man insgesamt 147,7 g 98,5%igen n-Butylester (GC), d. s. 84,0% d. Theorie; $Kp_{0,4\ mbar}$ = 113 bis 114° C.

## Beispiel 6

### N-Formylalaninisoamylester

106,3 g auf 0°C gebrachtes 65,9% $\alpha$-Alaninnitril (1 mol enthaltend 4,1% $NH_3$/0,26 mol) werden einer Lösung von 95,4 g HCl (= 2,6 mol) in 269,4 g (= 3 mol) iso-Amylalkohol wie im Beispiel 1 beschrieben zugesetzt, und innerhalb 2 Stunden auf 115°C aufgeheizt. Man läßt in 1 Stunde 59 g Formamid (= 1,3 mol) zulaufen und 2 Stunden am Rückfluß (120°C) sieden. Nach dem Abkühlen auf 60° werden 363 g Wasser zugesetzt und 388,5 g organische Phase abgetrennt, die nach 2maliger Wäsche mit je 40 g 3,5%iger Sodalösung i. V. fraktioniert wird. Man erhält 162 g 97%igen Ester (GC), d. s. 84,0% d. Th.; $Kp_{0,4\,mbar}$ = 120 bis 124°C.

## Beispiel 7

### N-Formyl-$\alpha$-aminobuttersäureisobutylester

103,4 g auf 0°C gebrachtes wäßriges 81,2%iges $\alpha$-Aminobuttersäurenitril (= 1 mol, enthaltend 0,25% $NH_3$/0,015 mol) werden einer 20°C messenden Lösung von 81,7 g HCl (= 2,24 mol) in 222 g Isobutanol (= 3 mol) wie im Beispiel 1 beschrieben zugesetzt, in 2 Stunden auf 104° gebracht, 3,5 Stunden am Rückfluß gehalten und dann mit 59 g Formamid (= 1,3 Mol) umgesetzt. Nach Wasserzugabe, Phasentrennung, Wäsche und fraktionierter Destillation erhält man 162,1 g 99%igen Ester (GC), d. s. 85,8% d. Th.; $Kp_{0,5\,mbar}$ = 125 bis 127°C.

## Beispiel 8

### N-Formylnorleucinisobutylester

136,6 g auf 0°C gebrachtes 82%iges $\alpha$-Aminocapronsäurenitril (entsprechen 1 mol Nitril mit 13,5% Wasser/1,02 mol/und 0,5% Ammoniak/0,04 mol werden mit einer Lösung von 87,4 g HCl/2,4 mol in 222 g Isobutanol/3 mol) wie im Beispiel 1 beschrieben zunächst für sich und dann mit 59 g Formamid (= 1,3 Mol) umgesetzt. Nach der Aufarbeitung erhält man 375 g organische Phase, die nach der Fraktionierung insgesamt 180,6 g N-Formylnorleucinisobutylester entsprechend einer Ausbeute von 84% d. Th. liefert. $Kp_{0,3\,mbar}$ = 131 bis 132°C.

## Beispiel 9

### N-Formylleucinethylester

122,5 g auf 0°C gebrachtes wäßriges 78,2%iges Leucinnitril (0,855 mol mit 0,6% Ammoniak/0,04 mol) werden wie im Beispiel 1 beschrieben mit einer Lösung von 85,5 g HCl (= 2,34 mol) in 184 g Ethanol (= 4,0 mol) umgesetzt, innerhalb von 3 Stunden auf 82°C gebracht und 4 Stunden bei dieser Temperatur gehalten. Man setzt innerhalb 1 Stunde 59 g Formamid (= 1,3 mol) zu und läßt bei 120°C 2 Stunden im verschlossenen Gefäß bei dem sich einstellenden Druck (5,4 bar) nachreagieren. Bei 60°C werden 363 g Wasser zugesetzt. Man erhält 230 g organische Phase, die nach der üblichen Aufarbeitung insgesamt 105 g 96%igen Ester liefert, d. s. 63% d. Th.;$Kp_{0,5\,mbar}$ = 107 bis 109°C. Die scheinbar geringe Ausbeute wird durch die Tatsache bedingt, daß das Veresterungsmittel (Ethanol) als Lösungsvermittler zwischen den Phasen wirkt. Aus der wäßrigen Phase lassen sich daher weitere Mengen an Formylester z. B. durch Extraktion gewinnen.

## Beispiel 10

### N-Formylleucinisobutylester

59,7 g auf 0°C gebrachtes wäßriges 82,1%iges Leucinnitril (0,438 mol enthaltend 0,7% Ammoniak/0,025 mol) werden mit einer Lösung von 42 g HCl (= 1,15 mol) in 111 g Isobutanol (= 1,5 mol) wie im Beispiel 1 beschrieben zunächst für sich und dann mit 29,5 g Formamid (= 0,65 mol) umgesetzt. Nach der üblichen Aufarbeitung erhält man 180 g gewaschene organische Phase, die nach fraktionierter Destillation insgesamt 87 g 98,8%igen N-Formylleucinisobutylester liefert, entsprechend einer Ausbeute von 91,3% d. Th.; $Kp_{0,4\,mbar}$ = 122 bis 125°C.

Beispiel 11

N-Formyl-methoxi-α-alaninisobutylester

121 g auf 0° C gebrachtes 82,6%iges Methoxi-α-alaninnitril (= 1,0 mol enthaltend 0,53% Ammoniak/ 0,038 mol) werden mit einer Lösung von 82 g HCl (= 2,25 mol) in 222 g Isobutanol (= 3,0 mol) und mit 59,5 g Formamid (= 1,3 mol) wie im Beispiel 1 beschrieben umgesetzt. Nach der Aufarbeitung erhält man 370 g organische Phase, die nach der destillativen Fraktionierung 160,4 g N-Formylmethoxi-α-alaninisobutylester liefert; Ausbeute: 79% d. Th.; $Kp_{0,1\ mbar}$ = 110° C, Fp. 28 bis 30° C.

Beispiel 12

N-Formyl-methionin-isobutylester

82 g auf 0° C gebrachtes 79,4%iges Methioninnitril (= 0,5 mol enthaltend 0,7% Ammoniak/0,03 mol) werden zu einer Lösung von 42,5 g HCl (= 1,16 mol) in 111 g Isobutanol (= 1,5 mol) unter Kühlen so zudosiert, daß eine Temperatur von 30° C nicht überschritten wird. Man erhöht innerhalb von 12 Stunden stufenweise die Temperatur bis auf 60° C, läßt dann 29,5 g Formamid (= 0,65 mol) zulaufen und erhitzt nach 3 Stunden unter Rückfluß bis auf 117° C. Nach der üblichen Aufarbeitung (vgl. Beispiel 1) erhält man 380 g organische Phase, die nach destillativer Fraktionierung 81,6 g N-Formyl-methionin-isobutylester liefert; Ausbeute: 70% d. Th., $Kp_{0,3\ mbar}$ = 155 bis 160° C.

**Patentansprüche**

1. Verfahren zur Herstellung von N-Formyl-α-aminosäureestern der Formel (V)

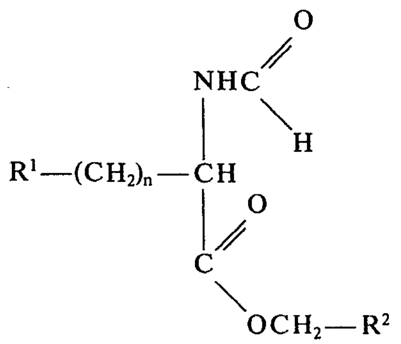

(V)

wobei n den Wert 0 oder 1 und $R^1$ Wasserstoff oder, falls n = 1 ist, auch einen Alkylrest mit bis zu 4 C-Atomen, die Methoxygruppe oder die Methylthiomethylengruppe bedeutet und $R^2$ eine Alkylgruppe von bis zu 5 C-Atomen darstellt, dadurch gekennzeichnet, daß man ein Aminonitril der Formel (I)

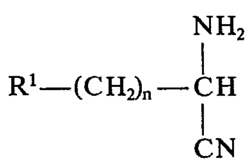

(I)

in der $R^1$ und n die vorstehende Bedeutung hat, mit einem entsprechenden Alkohol in Gegenwart von Chlorwasserstoff und von mindestens soviel Wasser, wie bei der Bildung des Aminonitrils aus seinem Cyanhydrin anfällt, umsetzt und das Umsetzungsprodukt mit überschüssigem Formamid bei bis zu 160° C umsetzt.

2. Verfahren nach Anspruch 1, dadurch gekennzeichnet, daß man die n-Propyl-, n-Butyl- oder Isobutylester herstellt.

3. Verfahren nach Anspruch 1, dadurch gekennzeichnet, daß man das Umsetzungsprodukt aus Aminonitril und Alkohol mit Formamid bei über 100° C umsetzt.

4. Verfahren nach Anspruch 2, dadurch gekennzeichnet, daß man α-Alaninnitril einsetzt.

**Claims**

1. A process for the preparation of N-formyl-$\alpha$-aminoacid esters of the formula (V)

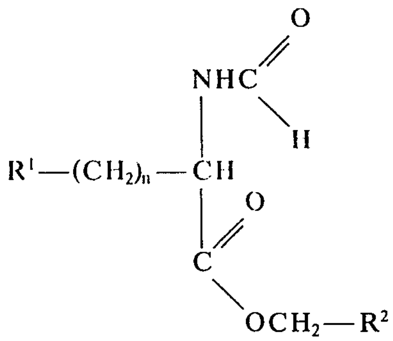

(V)

where n is 0 or 1 and $R^1$ is hydrogen or, if n is 1, may also be alkyl of up to 4 carbon atoms, methoxy or methylthiomethylene, and $R^2$ is alkyl of up to 5 carbon atoms, wherein an aminonitrile of formula (I)

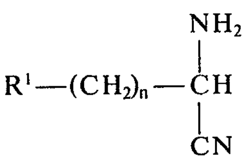

(I)

where $R^1$ and n have the above meanings, is reacted with a corresponding alcohol in the presence of hydrogen chloride and of at least the amount of water which arises on formation of the aminonitrile from its cyanohydrin, and the reaction product is reacted with excess formamide at up to 160° C.

2. A process as claimed in claim 1, wherein the n-propyl, n-butyl or isobutyl ester is prepared.

3. A process as claimed in claim 1, wherein the reaction product of aminonitrile and alcohol is reacted with formamide at above 100° C.

4. A process as claimed in claim 2, wherein $\alpha$-alaninonitrile is employed.

**Revendications**

1. Procédé pour la préparation d'esters de N-formyl-$\alpha$-aminoacides de formule (V)

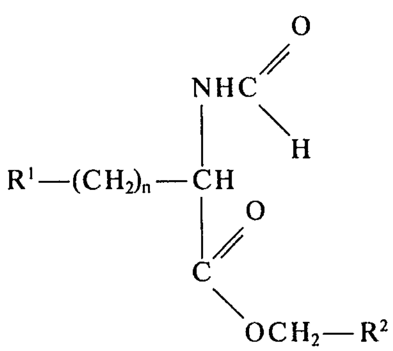

(V)

dans laquelle n a la valeur 0 ou et $R^1$ représente un atome d'hydrogène ou, au cas où n = 1, également un radical alkyle contenant jusqu'à 4 atomes de C, le groupe méthoxy ou le groupe méthylthiométhylène, $R^2$ représentant un groupe alkyle contenant jusqu'à 5 atomes de C, caractérisé en ce qu'on fait réagir un aminonitrile de formule (I)

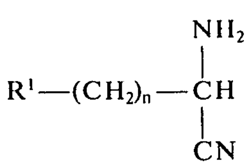

(I)

dans laquelle $R^1$ et n ont les significations données ci-dessus, avec un alcool approrié en présence d'acide chlorhydrique et d'au moins autant d'eau qu'il en est formé lors de la formation de l'aminonitrile à partir de sa cyanhydrine, et on fait réagir le produit de réaction avec du formamide en excès, à une température qui peut aller jusqu'à 160° C.

2. Procédé selon la revendication 1, caractérisé en ce qu'on prépare les esters n-propyliques, n-butyliques ou isobutyliques.

3. Procédé selon la revendication 1, caractérisé en ce qu'on fait réagir le produit de réaction de l'aminonitrile et de l'alcool avec le formamide à plus de 100° C.

4. Procédé selon la revendication 2, caractérisé en ce qu'on met en réaction l'$\alpha$-alanine-nitrile.